# EUROPÄISCHE PATENTSCHRIFT

(11) **EP 0 556 561 B1**
(45) Veröffentlichungstag und Bekanntmachung des Hinweises auf die Patenterteilung: **21.05.1997**
(21) Anmeldenummer: 93100531.8
(22) Anmeldetag: 15.01.1993
(51) Int. Cl.: A61M 16/08, F16L 53/00, H05B 3/58

(54) **Beheizbarer Beatmungsschlauch**
Heated respiratory hose
Tuyau chauffant pour ventilation respiratoire

(30) Priorität: 18.01.1992 DE 9200567 U; 30.12.1992 DE 4244493
(43) Veröffentlichungstag der Anmeldung: 25.08.1993
(73) Patentinhaber: EILENTROPP KG, D-51676 Wipperfürth (DE)
(72) Erfinder: Eilentropp, Heinz, W-5272 Wipperfürth (DE)

(56) Entgegenhaltungen:
- EP-A- 0 201 985
- FR-A- 2 599 115
- US-A- 4 121 583
- US-A- 4 214 147
- US-A- 4 682 010

## Beschreibung

Die vorliegende Erfindung betrifft einen Beatmungsschlauch aus einem durchscheinenden oder durchsichtigen gummielastischen Material mit an der äußeren Schlauchoberfläche wendelförmig verlaufenden Rippen oder Stegen, der durch elektrische Heizleiter beheizbar ist.

Beatmungsschläuche der gattungsgemäßen Art sind insbesondere in der Unfallmedizin ständig im Einsatz, sie dienen vor allem in Notfällen dazu, die Patienten mit der notwendigen Atmungsluft zu versorgen. Solche Schläuche sind durchsichtig oder zumindest durchscheinend, um eine ständige Kontrolle der Luftzufuhr durch das Bedienungspersonal sicherzustellen. Die an der Oberfläche angeordneten Rippen oder Stege dienen als mechanischer Schutz dazu, auch im geknickten Zustand des Schlauches noch einen ausreichenden Durchtrittsquerschnitt für die Atemluft über die Schlauchlänge zu gewährleisten. Beatmungsschläuche sind insbesondere für Notfälle vorgesehen, d. h. es ist nicht vorhersehbar, welche äußeren Rahmenbedingungen z. B. am Unfallort anzutreffen sind. Das gilt insbesondere auch für die dort herrschenden Witterungsbedingungen, so daß es bei entsprechend niedrigen Außentemperaturen immer wieder zu einem Beschlagen der Beatmungsschläuche an der inneren Oberfläche kommt. Das hierbei gebildete Kondenswasser kann vom Patienten eingeatmet werden und gelangt damit unmittelbar in die Lunge.

Bekannt ist ein Inhalationsapparat (EP 0 201 985 A1), der ein gewelltes Schlauchstück enthält, in dessen Wellentäler Heizkabel zur Beheizung des Schlauchstückes eingelegt sind. In diesem bekannten Heizkabel sind der Heizleiter, der Kaltleiter sowie ein zur Rundung des Kabels dienender Profilstrang gemeinsam geführt und von einer allen drei Elementen gemeinsamen Hülle umgeben.

Bekannt ist es auch bereits (EP 0 214 976 B1), zur Temperierung der Atemluft in den Hohlraum eines flexiblen Kunststoffschlauches mit glatter Innenwand einen Heizdraht bestimmten Widerstandes lose einzulegen. Abgesehen davon, daß ein solcher Kunststoffschlauch keine Gewähr bietet, daß im geknickten Zustand noch ein ausreichender Schlauchquerschnitt zum Transport der zugeführten Luft zur Verfügung steht, erfolgt an der nicht aufgeheizten Schlauchwandung eine nicht kontrollierbare Abkühlung der Atemluft. Um hier Abhilfe zu schaffen, ist bereits vorgesehen, den oder die Heizdrähte in den Spiralen des Atemluftschlauches anzuordnen. Bei einer solchen Anordnung ist die Wärmeverteilung über den Rohrquerschnitt nicht gleichmäßig, abgesehen davon, daß durch die Anordnung der Heizdrähte in den Spiralen des Atemluftschlauches eine große Isolierstrecke bis zum Schlauchinnern überbrückt werden muß. Hinzukommt, daß eine erhebliche Wärmemenge wegen der von der Schlauchoberfläche abstehenden Spiralen an die umgebende Außenluft abgeführt wird und damit nicht mehr zur Lösung der eigentlichen Aufgabe, nämlich der Temperierung der Atemluft im Innern des Schlauches, beiträgt.

Ausgehend von diesem bekannten Stand der Technik liegt der Erfindung die Aufgabe zugrunde, eine Möglichkeit zu finden, den Beatmungsvorgang unabhängig von den äußeren Witterungsbedingungen für den Patienten gefahrlos durchzuführen, d. h., für eine gleichmäßige Klimatisierung - Erwärmung bei natürlicher Feuchtigkeit - der Atemluft über den gesamten Schlauchquerschnitt zu sorgen, sowie den Beatmungsschlauch unabhängig von den äußeren Rahmenbedingungen stets betriebssicher zu halten.

Gelöst wird diese Aufgabe gemäß der Erfindung dadurch, daß die elektrischen Heizleiter isoliert und ein- oder beidseitig von den wendelförmigen Rippen oder Stegen sowie getrennt von einem Kaltleiter zur Stromzuführung unmittelbar auf der äußeren Schlauchoberfläche angeordnet sind. Damit ist auch bei niedrigen Außentemperaturen ein Beschlagen der inneren Rohrwandung durch Abkühlen des Atem- oder Beatmungsgases und damit eine Kondenswasserbildung innerhalb der dem Patienten zugeführten Beatmungsluft vermieden, dennoch kann das Atemgas gleichmäßig feucht gehalten werden, etwa durch Einstellung des Sättigungsgrades mit Hilfe sterilisierten Wassers. Neben der gleichmäßig feuchten, jedoch kondenswasserfreien Führung der Beatmungsluft kommt es aber nach wie vor darauf an, daß der Beatmungsschlauch trotz seiner Beheizung hochflexibel und mindestens durchscheinend ist, um fortlaufend eine Kontrolle über den Innenraum des Schlauches zu gewährleisten. Dies schon deshalb, weil oft Abscheidungen des Patienten in den Beatmungsschlauch gelangen und dort ebenfalls zu einer Gefährdung des Patienten, z. B. bei einer Notbeatmung, führen können. Sind, wie nach der Erfindung vorgesehen, die isolierten Heizleiter auf der äußeren Oberfläche des Beatmungsschlauches unmittelbar angeordnet, dann ist es nicht erforderlich, die Masse des Schlauchmaterials aufzuheizen, sondern es reicht in der Regel für die Zwecke der Erfindung aus, für eine hinreichende Abschirmung der Umgebungsluft zu sorgen.

Voraussetzung für die problemlose Handhabung eines Beatmungsschlauches ist auch, wie bereits ausgeführt, daß die Wandungen des Schlauches durchscheinend oder durchsichtig sind. Durch die entsprechend der Erfindung ein- oder beidseitig von den wendelförmigen Rippen oder Stegen angeordneten Heizleiter ist stets ein freier Einblick in das Schlauchinnere gewährleistet. Daran ändert sich auch nichts, wenn nach einem weiteren Erfindungsgedanken die Heizleiter auf der äußeren Schlauchoberfläche durch Kleben befestigt sind. In Anlehnung an das Schlauchmaterial, das vorzugsweise ein Silikonkautschuk ist, läßt sich das Ankleben der Heizleiter an der Schlauchoberfläche mittels eines entsprechenden Silikonklebers problemlos durchführen.

Durch die Isolierung der Heizleiter ist die mechanische Festigkeit auch bei starken Biegebeanspruchungen des Schlauches sichergestellt, abgesehen von der erhöhten Spannungsfestigkeit des umgebenden Isoliermaterials. Als zweckmäßig hat es sich auch erwiesen, wenn die Isolierung der Heizleiter aus einem Fluorpolymeren hergestellt ist. Die Verwendung dieser Isoliermaterialien, aber auch der Einsatz von Silikonkautschuk als Schlauchmaterial, als Material für die Rippen oder Stege und als Klebwerkstoff für die Rippen oder Stege und die Heizleiter erlaubt darüberhinaus eine Heißdampfsterilisation des erfindungsgemäßen Schlauches nach jeder Benutzung.

Die Rippen oder Stege, die den Beatmungsschlauch in Form einer Wendel umgeben, können aus einem massiven Silikonkautschukmaterial hergestellt sein, entscheidend ist, daß auch bei scharfen Biegungen diese Rippen oder Stege Stützfunktion auf die Schlauchwandung ausüben können, so daß in jedem Fall ein ausreichender Durchtritt der Beatmungsluft durch den Schlauchquerschnitt gewährleistet ist. Im Falle des beheizbaren Beatmungsschlauches kann in den als Vollprofil ausgebildeten Rippen oder Stegen in Weiterführung der Erfindung z. B. ein sog. Kaltleiter zur Stromzuführung zu dem oder den Heizleitern eingebettet sein. Als besonders vorteilhaft hat es sich jedoch in Durchführung der Erfindung erwiesen, die Rippen oder Stege als Schläuche auszubilden, so daß das Schlauchinnere beispielsweise zur Führung des Kaltleiters verwendet werden kann. Damit ergeben sich weitere Vorteile für die Erfindung. Einmal ist auch durch diesen in den Rippen oder Stegen mitgeführten Kaltleiter keine Sichtbehinderung in das Schlauchinnere gegeben, zum anderen läßt diese Anordnung auch zusätzliche schaltungstechnische Maßnahmen zu, beispielsweise für die Heizleiter, die hintereinander oder parallel geschaltet werden können, je nach den Bedingungen, die an den Beatmungsschlauch hinsichtlich der Heizleistung und damit Schirmwirkung oder hinsichtlich der Schlauchlänge gestellt werden. Ist, wie auch vorgesehen, der Kaltleiter lose im Innern der schlauchförmigen Rippen oder Stege geführt, dann trägt diese Maßnahme weiterhin dazu bei, den Beatmungsschlauch außerordentlich flexibel zu gestalten, um die für den Patienten günstigste Lage während der Fremdbeatmung auszuwählen. Das gesonderte Schlauchprofil ist vorzugsweise durch Kleben, beispielsweise mit Hilfe eines Silikonkautschuks, auf der Schlauchoberfläche befestigt.

In Weiterführung der Erfindung weist die Wendel der Rippen oder Stege eine flache Steigung auf, wobei der axiale Abstand zwischen je zwei aufeinanderfolgenden Windungen etwa der Breite jeder Rippe oder jedes Steges entspricht. Dies führt zu einer weiteren Stabilisierung des Schlauchquerschnittes auch bei scharfen Biegungen, außerdem führen beidseitig der Rippen oder Stege angeordnete elektrische Heizleiter oder Heizleitungen in diesem Fall zu einer besonders gleichmäßigen Temperierung der Schlauchoberfläche bzw. des Schlauchinneren.

Sind die elektrischen Heizleiter oder die Heizleitungen beiderseits der Rippen oder Stege angeordnet, dann können diese, falls es sich um kurze Schlauchlängen handelt, durch Verbindung an einem Ende elektrisch hintereinander geschaltet sein, wobei am anderen Ende des Schlauches die Heizleiterenden mit einer geeigneten Spannungsquelle verbunden sind. Bei größeren Schlauchlängen oder - querschnitten wird man die Heizleiter oder Heizleitungen in der Regel elektrisch parallel schalten, um so die Heizleistung zu erhöhen. In den Rippen oder Stegen kann dann der Kaltleiter mit geführt sein, der nach einem weiteren Erfindungsgedanken zusammen mit den Heizleitern an einem Ende des Beatmungsschlauches in Form einer Anschlußleitung herausgeführt ist. Dies führt zu einer wesentlichen Kompaktheit des Beatmungsschlauches selbst, der Schlauch ist damit problemlos zu bedienen, das gilt selbst dann, wenn in Notfällen wenig Zeit bleibt, um sich um den funktionsgerechten Anschluß der Heizleiter zu kümmern. Eine vorbestimmte Länge ist nämlich vorteilhaft in Durchführung der Erfindung durch beidseitige Schlauchanschlußstutzen sowie eine für Kaltleiter und Heizleiter gemeinsame elektrische Anschlußleitung konfektioniert.

In Weiterführung der Erfindung ist in diesem Zusammenhang an einem Schlauchende das eine Ende des Kaltleiters mit dem einen Ende der parallel geschalteten Heizleiter verbunden, während am anderen Schlauchende das andere Ende des Kaltleiters sowie die anderen Enden der Heizleiter zusammen in einer gemeinsamen elektrischen Anschlußleitung nach draußen geführt sind und in einem Anschlußstecker enden, der mit einer entsprechenden Spannungsquelle verbindbar ist.

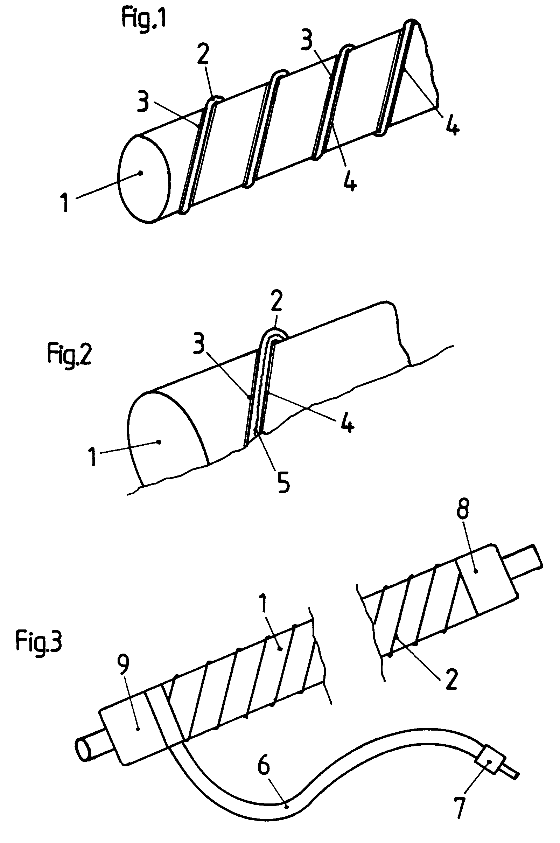

Die Erfindung sei anhand der in den Figuren 1 bis 3 dargestellten Ausführungsbeispiele näher erläutert.

Die Figur 1 zeigt einen hochflexiblen Beatmungsschlauch 1 nach der Erfindung, der beispielsweise aus einem Silikonkautschuk hergestellt ist und durchsichtig bzw. durchscheinend ist. An seiner äußeren Oberfläche verlaufen Rippen oder Stege 2 in Achsrichtung des Schlauches in Form einer Wendel mit möglichst flacher Steigung. Sie wirken als Knickschutz, wenn der Beatmungsschlauch 1 aus irgendwelchen Gründen scharf umgebogen wird. Unmittelbar am Fuße der Rippen oder Stege 2 verlaufen ebenfalls in Wendelform Heizleiter 3 und 4 beispielsweise als Litzenleiter, sie sind dicht an die Stege oder Rippen 2 herangeführt, so daß der zwischen den Rippen oder Stegen mögliche Durchblick in das Innere des Schlauches nicht behindert wird. Das gilt für die Heizleiter, Litzenleiter oder Massivdrähte, die mit einer dünnen Isolierung versehen sind.

Mitunter kann es ausreichen, auf die Heizleiter 4 zu verzichten und ausschließlich auf der einen Seite der Rippen oder Stege Heizleiter 3 anzuordnen. Ob dieser oder jener Lösung der Vorzug gegeben wird, hängt von der geforderten Heizleistung ab, die aufgebracht werden muß, um den Beatmungsschlauch gegen die äußere kalte Umgebungsluft abzuschirmen.

Die Figur 2 zeigt in einem gegenüber der Figur 1 vergrößerten Maßstab eine den Beatmungsschlauch umgebende Rippe 2, beidseitig zu dieser Rippe sind an ihrem Fußende auf der Schlauchoberfläche die Heizleiter 3 und 4 befestigt, die ebenfalls entsprechend der Wendel der Rippe 2 wendelförmig den Beatmungsschlauch 1 umgeben. Im Innern der Rippe 2, beispielsweise lose geführt, wenn es sich um ein Schlauchprofil handelt, ist der als Litzenleiter ausgebildete Kaltleiter 5 vorgesehen, er dient zur Stromhin- oder-rückführung, wenn z. B. die Heizleiter 3 und 4 zur Erhöhung der Heizleistung parallel geschaltet sind.

Die Figur 3 schließlich zeigt die erfindungsgemäße konfektionierte und daher universell einsetzbare kompakte Ausführungsform des beispielsweise ein bis drei Meter langen Beatmungsschlauches 1, der zur Abschirmung der kalten Außenluft sowie zur Klimatisierung der Beatmungsluft, d. h. Temperierung von z. B. auf 28° C bis 38° C und Befeuchtung durch geeignete Maßnahmen auf 100 % relative Feuchte oder wenig darunter, an seiner äußeren Oberfläche beheizbar ist. Die Heizung ist selbstverständlich regelbar, sie ist sowohl für den inspiratorischen als auch für den exspiratorischen Schlauchweg eines Beatmungssystems geeignet. Der Beatmungsschlauch selbst, etwa aus einem Silikonkautschuk, ist wiederum mit Rippen oder Stegen versehen, die diesen Schlauch in Achsrichtung wendelförmig umgebenden Rippen oder Stege sind mit 2 bezeichnet. Ein- oder beidseitig dieser Stege können wieder die Heizleiter oder Heizleitungen nach den Figuren 1 oder 2 angeordnet sein, im Steginneren ist ein Kaltleiter, wie aus der Figur 2 ersichtlich, mitgeführt, der dann zusammen mit den Heizleitern in Form der Anschlußleitung 6 nach außen geführt ist und in einem Anschlußstecker 7 endet. Beidseitig an den Enden des Beatmungsschlauches 1 sind Schlauchanschlußstücke 8 und 9 vorgesehen, sie dienen einmal zum Anschluß an das Beatmungsmundstück für den Patienten und zum anderen an das Beatmungsgerät.

## Patentansprüche

1. Beatmungsschlauch aus einem durchscheinenden oder durchsichtigen gummielastischen Material mit an der äußeren Schlauchoberfläche wendelförmig verlaufenden Rippen oder Stegen (2), der durch elektrische Heizleiter (3, 4) beheizbar ist, dadurch gekennzeichnet, daß die elektrischen Heizleiter (3, 4) isoliert und ein- oder beidseitig von den wendelförmigen Rippen oder Stegen (2) sowie getrennt von einem Kaltleiter (5) zur Stromzuführung unmittelbar auf der äußeren Schlauchoberfläche angeordnet sind.

2. Beatmungsschlauch nach Anspruch 1, dadurch gekennzeichnet, daß die Heizleiter (3, 4) auf der äußeren Schlauchoberfläche durch Kleben befestigt sind.

3. Beatmungsschlauch nach Anspruch 1 oder 2, dadurch gekennzeichnet, daß die Isolierung der Heizleiter aus Fluorpolymeren besteht.

4. Beatmungsschlauch nach Anspruch 1 oder einem der folgenden, dadurch gekennzeichnet, daß die Rippen oder Stege (2) zur Aufnahme eines Kaltleiters (5) dienen.

5. Beatmungsschlauch nach Anspruch 1 oder einem der folgenden, dadurch gekennzeichnet, daß die Rippen oder Stege (2) durch ein gesondertes Schlauchprofil gebildet sind, das den Schlauch (1) fortlaufend wendelförmig umgibt und auf der äußeren Schlauchoberfläche festgelegt ist.

6. Beatmungsschlauch nach Anspruch 5, dadurch gekennzeichnet, daß im gesonderten Schlauchprofil ein Kaltleiter (5) lose geführt ist.

7. Beatumungsschlauch nach Anspruch 1 oder einem der folgenden, dadurch gekennzeichnet, daß die Wendel der Rippen oder Stege (2) eine flache Steigung aufweist, wobei der axiale Abstand zwischen je zwei aufeinanderfolgenden Windungen etwa der Breite jeder Rippe oder jedes Steges (2) entspricht.

8. Beatmungsschlauch nach Anspruch 1 oder einem der folgenden, dadurch gekennzeichnet, daß der Schlauch (1) sowie die Rippen oder Stege (2) aus Silikonkautschuk bestehen.

9. Beatmungsschlauch nach Anspruch 8, dadurch gekennzeichnet, daß zur Befestigung der Heizleiter (3, 4) und/oder der Rippen oder Stege (2) auf der äußeren Schlauchoberfläche ein Silikonkautschuk dient.

10. Beatmungsschlauch nach Anspruch 1 oder einem der folgenden mit beidseitig der Rippen oder Stege (2) angeordneten Heizleitern (3, 4), dadurch gekennzeichnet, daß die längs der durch die Rippen oder Stege (2) gebildeten Wendel verlaufenden Heizleiter (3, 4) elektrisch parallel schaltbar sind.

11. Beatmungsschlauch nach Anspruch 1 oder einem der folgenden, dadurch gekennzeichnet, daß eine vorherbestimmte Länge durch beidseitige Schlauchanschlußstücke (8, 9) sowie eine für Kaltleiter (5) und Heizleiter (3, 4) gemeinsame elektrische Anschlußleitung (6) konfektioniert ist.

12. Beatmungsschlauch nach Anspruch 11, dadurch gekennzeichnet, daß an einem Schlauchende das eine Ende des Kaltleiters (5) mit dem einen Ende der parallel geschalteten Heizleiter (3, 4) verbunden ist, während am anderen Schlauchende das andere Ende des Kaltleiters (5) sowie die anderen Enden der Heizleiter (3, 4) zusammen in einer gemeinsamen elektrischen Anschlußleitung (6) nach draußen geführt sind und in einem Anschlußstecker (7) enden, der mit einer entsprechenden Spannungsquelle verbindbar ist.

## Claims

1. Breathing hose made of a translucent or transparent rubber elastic material with ribs or ridges (2) running helically on the outer surface of the hose and which can be heated by electric heating conductors (3, 4),
characterised in that the electric heating conductors (3, 4) are insulated and disposed on one or both sides of the helical ribs or ridges (2) and separate from a cold conductor (5) for supplying the current directly on the outer surface of the hose.

2. Breathing hose according to claim 1, characterised in that the heating conductors (3, 4) are secured on the outer surface of the hose by gluing.

3. Breathing hose according to claim 1 or 2, characterised in that the insulation of the heating conductors consists of fluoropolymers.

4. Breathing hose according to claim 1 or one of the following claims, characterised in that the ribs or ridges (2) serve to accommodate a cold conductor (5).

5. Breathing hose according to claim 1 or on of the following claims, characterised in that the ribs or ridges (2) are formed by a separate hose profile which continuously surrounds the hose (1) helically and is fixed on the outer surface of the hose.

6. Breathing hose according to claim 5, characterised in that a cold conductor (5) is guided loosely in the separate hose profile.

7. Breathing hose according to claim 1 or one of the following claims, characterised in that the helix of the ribs or ridges (2) exhibits a-shallow pitch, the axial distance between two successive turns roughly corresponding to the breadth of each rib or each ridge (2).

8. Breathing hose according to claim 1 or one of the following claims, characterised in that the hose (1) and the ribs or ridges (2) consist of silicone rubber.

9. Breathing hose according to claim 8, characterised in that a silicone rubber serves for fixture of the heating conductors (3, 4) and/or the ribs or ridges (2) on the outer surface of the hose.

10. Breathing hose according to claim 1 or one of the following claims with heating conductors (3, 4) disposed on both sides of the ribs or ridges (2), characterised in that the heating conductors (3, 4) running along the helix formed by the ribs or ridges (2) can be connected electrically in parallel.

11. Breathing hose according to claim 1 or one of the following claims, characterised in that a predetermined length is produced by hose connectors (8, 9) on both sides and a common electrical connecting lead (6) for the cold conductor (5) and the heating conductors (3, 4).

12. Respirator hose according to claim 11, characterised in that at one end of the hose one end of the cold conductor (5) is connected to one end of the heating conductors (3, 4) connected in parallel, while at the other end of the hose the other end of the cold conductor (5) and the other ends of the heating conductors (3, 4) are run out together in a common electrical connecting lead (6) and terminate in a connecting plug (7) which can be connected to a corresponding source of voltage.

## Revendications

1. Tuyau pour ventilation respiratoire en un matériau de gomme élastique translucide ou transparente avec sur la surface extérieure du tuyau des nervures ou des ailettes (2) en forme d'hélice, tuyau que l'on peut chauffer au moyen de conducteurs chauffants électriques (3, 4),
caractérisé en ce que
les conducteurs électriques chauffants (3, 4) sont isolés et sont disposés d'un côté ou des deux côtés des nervures ou des ailettes (2) en forme d'hélice directement sur la surface extérieure du tuyau en étant séparés par un thermistor servant à faire passer le courant.

2. Tuyau pour ventilation respiratoire selon la revendication 1,
caractérisé en ce que
les conducteurs chauffants (3, 4) sont fixés sur la surface extérieure du tuyau par collage.

3. Tuyau pour ventilation respiratoire selon la revendication 1 ou 2,
caractérisé en ce que
l'isolation des conducteurs chauffants est réalisée en un polymère fluoré.

4. Tuyau pour ventilation respiratoire selon la revendication 1 ou l'une des suivantes,
caractérisé en ce que
les nervures ou les ailettes (2) servent à recevoir un thermistor (5).

5. Tuyau pour ventilation respiratoire selon la revendication 1 ou l'une des suivantes,
caractérisé en ce que
les nervures ou les ailettes (2) sont formées par un profilé particulier, qui entoure le tuyau (1) de façon continue en forme d'hélice et est fixé sur la surface extérieure du tuyau.

6. Tuyau pour ventilation respiratoire selon la revendication 5,
caractérisé en ce que
on fait passer de façon lâche dans le profilé particulier un thermistor (5).

7. Tuyau pour ventilation respiratoire selon la revendication 1 ou l'une des suivantes,
caractérisé en ce que
l'hélice des nervures ou des ailettes (2) présente une pente aplatie, la distance axiale entre respectivement deux spires consécutives correspondant à peu près à la largeur de chaque nervure ou de chaque ailette (2).

8. Tuyau pour ventilation respiratoire selon la revendication 1 ou l'une des suivantes,
caractérisé en ce que
le tuyau (1) ainsi que les nervures ou les ailettes (2) sont en caoutchouc au silicone.

9. Tuyau pour ventilation respiratoire selon la revendication 8,
caractérisé en ce que
pour fixer les conducteurs chauffants (3, 4) et/ou les nervures ou les ailettes (2) sur la surface extérieure du tuyau on utilise un caoutchouc au silicone.

10. Tuyau pour ventilation respiratoire selon la revendication 1 ou l'une des suivantes, avec les conducteurs chauffants (3, 4) disposés des deux côtés des nervures ou ailettes (2),
caractérisé en ce que
l'on peut brancher électriquement en parallèle les corps chauffants (3, 4) qui s'étendent le long de l'hélice formée par les nervures ou les ailettes (2).

11. Tuyau pour ventilation respiratoire selon la revendication 1 ou l'une des suivantes,
caractérisé en ce que
l'on confectionne une longueur déterminée auparavant par des pièces (8, 9) de raccordement au tuyau des deux côtés et une ligne électrique de raccordement (6) commune au thermistor (5) et aux conducteurs chauffants (3, 4).

12. Tuyau pour ventilation respiratoire selon la revendication 11,
caractérisé en ce que
l'on relie à l'une des extrémités du tuyau l'une des extrémités du thermistor (5) à l'une des extrémités des conducteurs chauffants (3, 4) montés en parallèle, tandis qu'à l'autre extrémité du tuyau ont fait passer ensemble l'autre extrémité du thermistor (5) ainsi que les autres extrémités des conducteurs chauffants (3, 4) dans une ligne électrique commune (6) de raccordement vers l'extérieur, extrémités qui se terminent dans un connecteur (7), qui peut être relié à une source de tension correspondante.
